# EUROPEAN PATENT SPECIFICATION

(11) **EP 4 376 906 B1**
(45) Date of publication and mention of the grant of the patent: **11.03.2026**
(21) Application number: 22760682.9
(22) Date of filing: 29.07.2022
(51) Int. Cl.: A61L 27/06, A61L 27/34, A61L 27/46, A61L 27/56, A61L 31/02, A61L 31/10, A61L 31/12, A61L 31/14, C01B 25/32

(54) **POROUS HYDROPHILIC COMPOSITES FOR USE IN PROMOTING BONE GROWTH**
PORÖSE HYDROPHILE VERBUNDMATERIALIEN ZUR FÖRDERUNG DES KNOCHENWACHSTUMS
COMPOSITES HYDROPHILES POREUX DESTINÉS À FAVORISER LA CROISSANCE OSSEUSE

(30) Priority: 30.07.2021 GB 202111039
(43) Date of publication of application: 05.06.2024
(73) Proprietor: Promimic AB, 431 53 Mölndal (SE)
(72) Inventor: KJELLIN, Per, 431 83 Mölndal (SE); VIKINGSSON, Line, 431 83 Mölndal (SE); FATHALI, Hoda, 431 83 Mölndal (SE); SIMONARSON, Gunnar, 431 83 Mölndal (SE); DAVIDSSON, Oscar, 431 83 Mölndal (SE)
(74) Representative: Dehns
(86) International application number: PCT/EP2022/071400
(87) International publication number: WO 2023/006969

(56) References cited:
- US-A1- 2011 111 004
- KIM HAE-WON: "Biomedical nanocomposites of hydroxyapatite/polycaprolactone obtained by surfactant mediation", JOURNAL OF BIOMEDICAL MATERIALS RESEARCH PART A, vol. 83A, no. 1, 1 January 2007 (2007-01-01), US, pages 169 - 177, XP055908127, ISSN: 1549-3296, DOI: 10.1002/jbm.a.31247
- DUAN B ET AL: "Synthesis of Ca-P nanoparticles and fabrication of Ca-P/PHBV nanocomposite microspheres for bone tissue engineering applications", APPLIED SURFACE SCIENCE, ELSEVIER, AMSTERDAM, NL, vol. 255, no. 2, 15 November 2008 (2008-11-15), pages 529 - 533, XP025586260, ISSN: 0169-4332, [retrieved on 20080626], DOI: 10.1016/J.APSUSC.2008.06.057
- MOHD YUSOFF MOHD FAIZ ET AL: "Dipcoating of poly ([epsilon]-caprolactone)/hydroxyapatite composite coating on Ti6Al4V for enhanced corrosion protec", SURFACE AND COATINGS TECHNOLOGY, vol. 245, 1 March 2014 (2014-03-01), pages 102 - 107, XP028634069, ISSN: 0257-8972, DOI: 10.1016/J.SURFCOAT.2014.02.048
- MAKAROV C ET AL: "In situ synthesis of calcium phosphate-polycaprolactone nanocomposites with high ceramic volume fractions", JOURNAL OF MATERIALS SCIENCE: MATERIALS IN MEDICINE, KLUWER ACADEMIC PUBLISHERS, BO, vol. 21, no. 6, 9 March 2010 (2010-03-09), pages 1771 - 1779, XP019822075, ISSN: 1573-4838

## Description

### FIELD OF THE INVENTION

The present invention is defined by the appended claims and relates generally to a porous hydrophilic composite which can serve as a scaffold for bone cell growth and to processes to prepare it. The composite comprises nanosized calcium phosphate (CaP) and a biodegradable polymer. The composite can be applied to a surface, such as an implant surface. The thickness of the composite layer on the implant surface can be controlled.

### BACKGROUND OF THE INVENTION

In medicine, there are many situations where it is necessary to aid the human body to build new bone. Small fractures are normally healed without guidance, provided that the bone fracture ends are positioned sufficiently close to each other. Bone tissue is able to overbridge gaps of some millimetres, but bone loss above 5-10 mm is a so-called critical size defect which needs treatment to heal. Complex fractures may damage the bone tissue to such an extent that normal healing is insufficient. Tumours, which destroy large portions of bone tissue, can make it impossible for the body to heal the injury by itself. Another example is so called sinus lifts, where the natural bone is too thin to support a dental implant and the bone tissue has to be augmented.

The insertion of a bone scaffold is a common procedure to help the human body to create new bone. Bone scaffold materials contain a structure and composition which will trigger the formation of bone when implanted in the human body, and act as a cultivating substrate for bone cells.

Bone scaffolds can be of biological origin, for example harvested from the patient's own bone prior to the surgery (autograft), or from other donors (allograft). The bone scaffold may also be produced from animals, such as bovine bone (xenograft). Autografts and allografts have an excellent effect on bone tissue growth, but require surgery to remove the bone, and the handling and storage of the graft is complicated. A xenograft has the benefit of not requiring any patient surgery for the harvesting, but needs rigorous cleaning and sterilization procedures due to the risk of infection.

Synthetic bone scaffolds have also been proposed. The primary benefits of synthetic scaffolds compared to bone grafts of biological origin are that they can be made in large quantities and with controlled specifications. Synthetic bone scaffolds can be in the form of a powder, such as hydroxyapatite (HA) or tricalcium phosphate. Powder based products are successful in restoring bone tissue but are less suitable for load-bearing applications.

Another type of bone scaffold is the rigid type, which usually consists of porous HA or tricalcium phosphate which has been sintered at high temperatures. The primary advantages of rigid bone scaffold products are form stability when implanted and the porosity, which increases the available surface area for the new bone cells to grow on. One disadvantage is the brittleness of the structure; a rigid bone scaffold normally contains no flexible substances such as polymers, which may dissipate forces applied to the material and prevent crack formation.

One way of decreasing the brittle nature of a rigid mineral scaffold is to incorporate a polymer into the structure. Polymer/mineral composite materials consist of a polymer, which can be biodegradable, and a mineral with bone regeneration properties, such as HA. The polymer makes the composite elastic and crack-resistant, while the mineral induces the formation of endogenous bone.

In addition to the porosity of the bone scaffold, the crystal size of the calcium phosphate is also important to stimulate the growth of endogenous bone. HA, with the chemical formula Ca₅(PO₄)₃OH, is a mineral which closely resembles the calcium phosphate mineral found in natural bone. For certain biomaterial applications, it is highly desirable to use nanosized HA which is similar to the size and shape as in human bone, i.e. with a particle size of 1-100 nm in length. Natural bone consists of rod-shaped calcium phosphate crystals with a length of 20-40 nm, 2 nm thick and 2-4 nm wide, surrounded by a collagen network (H. A. Lowenstam and S. Weiner, On biomineralization, Oxford University Press, New York, 1989).

It is generally considered that the bioactivity of HA is improved if the HA crystals are of a similar size and shape as those produced by the human body. Nanocrystalline HA may promote bone remodelling since the body recognizes the nanosized HA as a part of its own bone tissue and starts to grow new bone around the foreign object. For implants, a coating with nanosized HA will significantly increase the bone cell activity compared to microsized HA. For polymer/HA composites, the bioactivity as well as the strength is greatly improved with nanosized HA (J. Wei, Y. Li and K. Lau, Composites part B: engineering, 2007, 38, 301-305; H. Ramay and M. Zhang, Biomaterials, 2003, 24, 3293-3302).

In the literature, the fabrication of a number of bioactive and biodegradable porous scaffolds made of polymers and ceramic materials, such as polycaprolactone and hydroxyapatite, have been described. For example, porous PCL and HA composites with different proportions of HA can be manufactured by selective laser sintering. The researchers found that the compressive stiffness increased with increasing amount of HA, and the porosity decreased (K. Rezwan et al. Biomaterials 2006 27(18): 3413-31). Another example is the fabrication of porous PCL/HA composites by precision extrusion, which produced scaffolds with different porosity and pore size. Osteoblasts were cultivated and shown to migrate and proliferate for these scaffolds (L. Shor et al. Biomaterials Volume 28, Issue 35, December 2007, Pages 5291-5297).

US2010/226956 describes a moldable composite made of polycaprolactone, hydroxyapatite and different bioresorable plasticizers. The ability to shape the composite is controlled by the amount and type of plasticizer used. Once the composite is implanted in the body, the plasticizers are resorbed to leave a porous structure. The composite is made by melt mixing. However, it has been found that the hydrophilic HA does not fully disperse in the hydrophobic polymer during the melt mixing, leading to a composite which contains large domains of pure polymer which render the overall composite hydrophobic (i.e. with a water contact angle >90 °). Also US 2011/111004 A1 discloses a porous osteoinductive composite material comprising a biocompatible polymer, such as PCL or PLA and calcium phosphate nanocrystals.

CN102008752A discloses a method of forming a nano-hydroxyapatite coating on a porous biphasic calcium phosphate biological scaffold using chemical precipitation. The result is a porous biphasic calcium phosphate bioscaffold with a nano-hydroxyapatite coating. However, the HA crystals are not dispersed in a polymer matrix.

EP3785743A presents various ways of producing porous polymer scaffolds with calcium phosphate content, however the calcium phosphate used in the scaffolds is not nanosized.

CN107823715A discloses a PCL/HA composite porous bone tissue engineering scaffold that is characterized in that the scaffold is composed of polycaprolactone and hydroxyapatite.

The content of hydroxyapatite is 0.5 to 50% by mass percentage, and the rest is polycaprolactone. The porosity is controlled by the use of pore formers such as sodium carbonate, sodium bicarbonate and potassium carbonate, and these compounds are subsequently removed by the use of strong acids, such as hydrochloric acid, sulphuric acid or nitric acid which dissolve the pore forming compounds. The formation of carbon dioxide during the dissolution creates pores and helps to create an interconnecting porous structure. However, the acids used to dissolve the pore forming agents will also dissolve any HA which is not fully embedded in and protected by the polymer matrix. The result will be a polymer/HA composite with little or no HA present on the surface of the polymer, which will decrease the hydrophilicity and biological activity towards bone cell growth. Additionally, since PCL starts to degrade at acidic pH, this method increases the risk of premature degradation of the PCL matrix.

US6165486A discloses a composition comprising HA, a polycaprolactone (PCL) and a copolymer of poly(lactic) acid and poly(glycolic) acid (PLGA) and molding the blend and bioceramic into an implant. The porosity is created by adding NaCl as the pore forming agent, which is removed with water. However, there is always a risk of having residual NaCl crystals present in the composite, which will have a negative effect on the performance *in vivo.* Weight loss curves for different composites stored at 37 °C in phosphate-buffered saline (pH 7.4) are shown in Figures 1 and 2 of US6165486A. Since PCL and PLGA are expected to degrade very slowly in a sterile PBS buffer at pH 7.4 without any enzyme present, it appears that the observed weight loss may be due to NaCl crystals added to create porosity which dissolve and decrease the weight, taking more than 8 weeks to leach out from the structure.

ES2330823A discloses a polymeric scaffold formed by thermally induced phase separation with hydroxyapatite deposition. The scaffold consists of a polymer network with a thin coating of HA on the internal surface of the pores in the polymer network.

CN100546661C discloses a method for preparing an in situ pore-forming self-setting calcium phosphate composite scaffold. The porous calcium phosphate scaffold is then filled with a polymer, which may then be cross-linked. The resulting product is a dense structure with low porosity and no open cells.

Bone scaffolds are often used together with a load-bearing, non-degradable structure, such as a spinal fusion cage. This approach is particularly effective when larger volumes of bone need to be built around the implant. In the case of spinal fusion surgery, the interior of the spinal fusion cage may be packed with a bone scaffold material, to combine the high mechanical strength of the spinal cage with the bone stimulation properties of the bone scaffold. However, this type of implant is fragile to handle, and requires preparation of the implant with the bone scaffold on site. It would therefore of great benefit to have a scaffold which can be tailor made for the implant.

### OBJECTS OF THE INVENTION

The present invention is defined by the appended claims. Herein disclosed is a porous hydrophilic composite, serving as a scaffold for bone cell growth, comprising of nanosized calcium phosphate, such as amorphous calcium phosphate (ACP), beta-TCP, calcium deficient HA (CDHA) or hydroxyapatite (HA), and a biodegradable polymer. Also disclosed is a method to produce a layer of the composite onto a surface, such as an implant surface, to form an osteoconductive coating on the implant. Further disclosed is a method to produce the porous composite in situ within a void of an implant or on the surface of an implant, or within the pores of a porous metal lattice structure, with the ability to control the geometry of the composite. Disclosed is also the ability to control the geometry by layer-by-layer deposition, to create a highly ordered structure.

Objects and advantages of the present invention will become obvious to the reader and it is intended that these objects and advantages are within the scope of the present invention.

### SUMMARY OF THE INVENTION

In one aspect, there is provided a porous hydrophilic composite which can serve as a scaffold for bone cell growth. The porous hydrophilic composite comprises:
(a) a porous biodegradable polymer matrix, and
(b) nanosized calcium phosphate (CaP) homogeneously dispersed throughout the polymer matrix, wherein the CaP has a specific surface area in the range of about 180 to about 380 m²/g;
(c) from about 0.5 to 50 wt% of a biocompatible organic polyol, based on the total weight of the composite including the biocompatible organic polyol;
with the proviso that the composite does not contain any copolymer(s) of lactic acid and glycolic acid.

In another aspect, there is provided a scaffold for use in bone reconstruction, the scaffold comprising the porous hydrophilic composite of the invention and a substrate, such as an implant. The composite may be present as a coating on a surface of the substrate, or the substrate may be wholly or partially surrounded by the composite.

In another aspect, there is provided a process for the preparation of a porous hydrophilic composite according to the invention the process comprising:
(a) mixing a solution of a biodegradable polymer in a first solvent with nanosized calcium phosphate (CaP), preferably with a dispersion of nanosized calcium phosphate (CaP) in a second solvent, until the CaP is homogeneously distributed throughout the resulting mixture;
(b) solidifying the mixture of step (a) to form a gel; and
(c) removing the first and second solvents from the gel of step (b) by (i) washing with a third solvent to leave a porous hydrophilic composite containing the nanosized CaP homogeneously dispersed in a porous polymer matrix, or
   (ii) by evaporation to leave a layer of a porous hydrophilic composite containing the nanosized CaP dispersed throughout a porous polymer matrix on the surface, wherein the process further comprises
(d) immersing the porous hydrophilic composite in a solution comprising a biocompatible organic polyol;
(e) removing the porous hydrophilic composite from the solution; and
(f) drying the porous hydrophilic composite.

In the processes of the invention, the dispersion of nanosized calcium phosphate (CaP) in a second solvent may be prepared by mixing a dispersion of a non-phosphate calcium salt such as CaO in a suitable solvent with an aqueous phosphoric acid solution.

In the processes of the invention, the first and second solvents may be the same or different, but are preferably the same. The third solvent used in the first process is different to the first and second solvents.

The processes of the invention further comprise (d) immersing the porous hydrophilic composite in a solution comprising a biocompatible organic polyol, (e) removing the porous hydrophilic composite from the solution and (f) drying the porous hydrophilic composite, to further increase the hydrophilicity of the composite structure.

The CaP used in the above methods preferably has a surface area in the range of about 180 to about 380 m²/g.

Disclosed herein is a method for the preparation of nanosized CaP, the method comprising mixing a dispersion of a non-phosphate calcium salt in a suitable non-aqueous solvent with an aqueous phosphoric acid solution to form amorphous nanosized CaP, and optionally contacting the amorphous CaP with water to convert it to HA or β-TCP.

### BRIEF DESCRIPTION OF THE FIGURES

Figure 1. Powder X-ray diffractogram of a nanocrystalline HA sample.
Figure 2. Powder X-ray diffractogram of a nanosized amorphous HA sample.
Figure 3a. Schematic of composite production, step 1.
Figure 3b. Schematic of composite production, step 2.
Figure 4. A porous composite, prepared according to Example 1d, without nanosized HA after immersion in toluidine blue (a) and the same sample cut in two pieces, showing the inside of the sample (b). A porous composite prepared according to Example 1c, with nanosized HA after immersion in toluidine blue (c) and the same sample cut in two pieces, showing the inside of the sample (d).
Figure 5. Illustration of spinal cage (left images) with a layer of composite, prepared according to Example 6, on the insides of the implant (right images).
Figure 6. Images of titanium lattice with in situ formed CaP / PCL foam, prior to immersion in Alizarin Red (a) and (b). Image of different 3D printed titanium samples with different mesh size, after immersion in Alizarin Red (c).
Figure 7. Illustration of method to produce a screw coated with a 1 mm layer of composite (a). Photo of screw coated with composite (b).
Figure 8. Micro-CT images of sham (a) after 6 weeks, and sham with porous composite in place after 6 weeks (b) placed in the calvaria of rabbits. The site with porous composite is completely overgrown with bone.
Figure 9. Masson-Goldner dye stained slide of the sample shown in Figure 8b.
Figure 10. Scanning Electron Microscope (SEM) image of the sample produced in Example 1c.
Figure 11. Image of pin array placed in foam according to Example 10.
Figure 12a. Photograph of the composite of Example 1c after soaking in red dye for 30 minutes.
Figure 12b. Photograph of the composite of Example 1c after glycerol treatment according to Example 11 and subsequent soaking in red dye for 30 minutes.

### DETAILED DESCRIPTION OF THE INVENTION AND PREFERRED EMBODIMENTS THEREOF

Reference will now be made in detail to the presently preferred embodiments of this invention.

Ranges obtained by combining any of the lower limits with any of the upper limits of ranges disclosed herein are also included in the present invention

The present invention comprises a porous hydrophilic composite comprising (or consisting essentially of or consisting of) nanosized calcium phosphate (CaP) and a matrix of a biodegradable polymer, as defined in the claims. Due to the bioactive properties of the nanosized CaP particles, the high surface area of the polymer / CaP composite and its hydrophilicity, the composite may serve as an excellent scaffold for bone cell growth.

### CaP

As used herein, nanosized refers to particles with a longest dimension of 1 to 100 nm. Preferred are particles with a longest dimension of 1 to 50 nm, for example 5-40 nm or 10-20 nm.

The particles may be of any shape, including substantially spherical, of hexagonal cross section or fibre shaped.

As used herein, CaP refers to calcium phosphate in any form, including but not limited to amorphous CaP (ACP), beta-tricalcium phosphate (β-TCP), tetracalcium phosphate (TTCP), hydroxyapatite (HA) or calcium deficient HA (CDHA). Preferred forms of CaP include HA.

Amorphous calcium phosphate (ACP) is non-crystalline and can have a varying Ca/P ratio, Beta-tricalcium phosphate (β-TCP) has a Ca/P ratio of 1.5. The dissolution of the calcium phosphate particles is greatly influenced by their structure and size. The resorption speed of calcium phosphate polymorphs in an acidic environment is ACP > β-TCP > TTCP > HA (S.V. Dorozhkin, Biomaterials 31 (2010) 1465-1485), and the dissolution and release of calcium and phosphorus *in vivo* can be controlled by the choice of CaP compound used.

The CaP may be amorphous or crystalline. For crystalline CaP, the term "nanosized" refers to the size of the individual crystals whereas for amorphous CaP it refers to the size of individual particles. Nanosized crystalline CaP may also be referred to as "nanocrystalline CaP".

If amorphous CaP with a Ca/P ratio of 5/3 is used in the composites of the invention, it may undergo conversion to crystalline HA when exposed to bodily fluids *in vivo.* Since amorphous CaP has a lower mechanical strength compared to crystalline HA, this effect can be used to create a composite which will increase in strength after implantation. In addition, since the transformation from ACP to HA is thermodynamically favourable, it will accelerate the infiltration of body liquids and also increase the adhesion strength between the composite and surrounding bone.

Nanosized CaP may be available commercially, usually in powder form, or may be synthesised using methods known in the art. For example, methods for the synthesis of nanosized CaP are disclosed in WO2005/123579 and US2010/0226956.

Nanosized crystalline CaP may synthesized by a method which involves the mixing of (i) a dispersion of a non-phosphate calcium salt in water or a non-aqueous solvent with (ii) an aqueous phosphoric acid solution. This results in a dispersion of nanocrystalline CaP which may be used directly in the first or second method to form a composite as discussed below.

By using a non-aqueous dispersion of a non-phosphate calcium salt instead of an aqueous dispersion, together with an optional further aging step, the method can be used to produce HA or other types of calcium phosphate such as ACP, beta-TCP or CDHA.

The ratio of non-phosphate calcium salt to phosphoric acid can influence the CaP compound formed.

To obtain CaP with a high surface area, the non-phosphate calcium salt should have a low solubility in water or in the solvent used to form the non-aqueous dispersion. Preferred non-phosphate calcium salts are calcium oxide and calcium hydroxide.

Disclosed is a method for the preparation of amorphous nanosized CaP, the method comprising mixing a dispersion of a non-phosphate calcium salt in a suitable non-aqueous solvent with an aqueous phosphoric acid solution to form amorphous nanosized CaP. Suitable non-aqueous solvents for use in this method include glycerol and ethylene glycol.

Preferably, the non-aqueous solvent is miscible with water. Preferably, the non-aqueous solvent is miscible with acetone or another volatile polar solvent to allow it to readily removed by washing with such a solvent.

Optionally, the CaP formed in this method can then be treated by contacting it with water to convert it into a different form of CaP. For example, if ethylene glycol or glycerol are used to form the dispersion of the non-phosphate calcium salt rather than water, ACP can be produced. The ACP can optionally be aged by contact with water at ambient temperature to produce HA. Alternatively, contact with water at elevated temperature and pressure, such as in an autoclave, can produce β-TCP. Autoclaving involves heating the powder in a sealed container (e.g. a stainless steel bomb) in the presence of water. Since this is done at elevated temperatures (e.g. 180-220 °C), the pressure will be above 1 atmosphere, typically between 5 and 15 atmospheres. The particle size and surface area of the CaP produced can be controlled by controlling the temperature and duration of the contact with water.

The specific surface area of the CaP is in the range of about 180 to about 380 m²/g as measured by the BET method (S. Brunauer, P.H. Emmet, E. Teller, J. Am. Chem. Soc. 1938, 60, 309-319*).* Preferably, the specific surface area of the CaP is in the range of about 200 to about 350 m²/g, more preferably in the range of about 200 to about 250 m²/g. Amorphous nanosized CaP will tend to have a higher surface area than nanocrystalline CaP.

X-ray diffraction (XRD) may also be used to give a rough measurement of the surface area of CaP in a composite. For more accurate measurement, the polymer may be dissolved from the composite using a suitable solvent (for example acetone) and the surface area of the CaP thus obtained may be measured by the BET method.

### Polymer

The polymer used in the composites should be biodegradable *in vivo,* preferably degrading over a period of about 3 months to about 3 years, for example about 6 months to about 2 years, preferably over about 3 to about 12 months.

The polymer used in the composites should also be biocompatible.

The biodegradable polymer may be synthetic or naturally occurring. Suitable biodegradable polymers include synthetic polyesters such as poly(caprolactone) (PCL), poly(lactic acid) (PLA), poly(glycolic acid) (PGL), poly(vinyl alcohol) (PVA), and naturally occurring polymers such as soluble collagen, hyaluronic acid, gelatine and chitosan. These polymers undergo hydrolysis in the human body, producing non-toxic degradation products (L. Nair and C. Laurencin, Progress in polymer science, 2007, 32, 762-798).

The biodegradable polymer may be water soluble or water insoluble, but water insoluble polymers are preferred. Polyesters such as PCL, PLA and PGL are water insoluble, whilst poly(vinyl alcohol) (PVA), soluble collagen, hyaluronic acid, gelatine and chitosan are water soluble.

The biodegradation speed can be governed by the choice of polymer. For example, PCL generally degrades more slowly than PLA or PGA *in vivo.*

A particularly preferred polymer for use herein is PCL. More preferably, PCL is the only polymer present in the composite. For example, the polymer matrix may consist of PCL. PLA and PGL can degrade *in vivo* to produce acidic degradation products (lactic acid and glycolic acid respectively) which may give rise to undesirable inflammatory reactions. In contrast, the degradation product of PCL *in vivo* (6-hydroxyhexanoic acid) is less likely to give rise to undesirable inflammatory reactions since it is less acidic; the pKa of lactic acid is 3.86 while the pKa of 6-hydroxyhexanoic acid is 4.75.

Preferably the polymer matrix is formed from a single polymer, although blends may also be used.

The composites do not include any copolymer(s) of lactic and glycolic acid, such as poly (D,L-lactic-co-glycolic) acid or a copolymer of poly(lactic) acid and poly(glycolic) acid. Preferably, the biodegradable polymer is a homopolymer (i.e. is not a copolymer). Preferably, the composites of the invention do not contain any copolymers.

### Composites

The composites of the present invention are porous once formed and before use *in vivo.* They have an open cell foam-like structure and may also be referred to as "foams".

Preferably, the pores in the composite comprise nanopores and micropores, where the nanopores are created by the nanosized CaP particles, and the micropores are created by the polymer network. The nanopores have a longest dimension of from about 1 to about 100 nm, and the micropores have the longest dimension of from about 1 to 100 µm.

The size of the nanopores may be measured using SEM, and/or by nitrogen adsorption and desorption using the Barett-Joyner-Halenda (BJH) method. The size of the micropores can be measured using SEM and/or by mercury porosimetry.

The porosity of the composites can be calculated by measuring the density of an experimental sample and comparing this with the theoretical density of a massive sample (i.e. a non-porous sample containing the same amounts of polymer and CaP). For example, if the density of PCL is 1.14 g/cm³, the density of HA is 3.15 g/cm and the composition has a PCL / HA ratio of 2:1, the theoretical density of this sample will be (2*1.14 + 3.15) / 3 = 1.81 g/ cm³. Thus, a massive cube (non-porous) with a side of 1 cm of this composition should weigh 1.81 g. If the actual weight of a cubic sample of this composition with a side of 1 cm is 0.181 g, then the porosity is 1-(0.181 / 1.81) = 90 %.

Preferably, the porous composites of the invention have a porosity in the range of about 20 to about 95%, more preferably in the range of about 50 to about 90 %, and even more preferably in the range of about 60 to about 90%. Whilst high levels of porosity are desirable to promote bone growth *in vivo,* highly porous composites will have low mechanical strength and will not be suitable for all applications.

The composite may further comprise additional voids created by removable supports. Such voids may have a longest dimension of about 5 to 40 mm. Voids can be introduced into the composite by solidifying the composite around a removable support, which when removed leaves voids in the composite. For example, the composite may be moulded around a removable pin array. Following solidification of the composite, the pin array may be removed, leaving voids in the composite corresponding to the shape and size of the pin array. The pins may have any cross-section but cylindrical pins are preferred. Suitable diameters for cylindrical pins are about 0.25 to about 2 mm.

The removable support may be made of metal, for example stainless steel. To aid removal of any removable support from the composite, the surface of the support should be smooth, for example a polished metal surface.

The presence of millimetre-sized voids in the composite may improve vascularization, i.e. growth of blood vessels, within the composite *in vivo,* for example when it implanted in a patient.

The composites of the invention are hydrophilic when manufactured and remain hydrophilic as the polymer matrix biodegrades *in vivo.* By hydrophilic is meant they have a contact angle with water of < 90°. The lower the contact angle, the more hydrophilic the composite is and the better it will be at promoting bone growth *in vivo.* Preferably, the composites have a contact angle with water of about 70° or below, more preferably of about 60° or below.

The contact angle of a sample can be measured by placing a drop of Type 1 water (resistivity 18.2 MΩ/cm) on a flat part of the sample and measure the contact angle between the drop and the surface with a goniometer.

When implanted, the porosity and hydrophilicity of the composites promote rapid infiltration and adsorption of bodily fluids, osteointegration and adherence of new bone cells. Over time, the polymer of the matrix will biodegrade and the CaP will dissolve, leading to the composite eventually being replaced by new bone tissue.

In the composites of the present invention, the nanosized CaP is homogeneously dispersed throughout the polymer matrix. As a result, some of the CaP will initially be completely surrounded by polymer (i.e. embedded within the polymer) and some will be exposed at the polymer surface, including at the surface of pores within the polymer matrix. The exposed CaP renders the composite as a whole hydrophilic even when a hydrophobic polymer such as PCL or a polymer with low hydrophilicity such as PLA is used to form the composite.

Previously known implants where the CaP is present as a surface coating on a polymeric structure will be hydrophilic initially but as the CaP is dissolved *in vivo,* the underlying polymer will be exposed. As polymers are much less efficient than CaP in promoting osteointegration, this can be detrimental to the growth of new bone. In contrast, in the present invention the CaP is homogeneously distributed throughout the polymer which forms the matrix. CaP at the surface of the polymer matrix renders the composite hydrophilic initially. As the polymer biodegrades *in vivo,* CaP which was previously surrounded by polymer will be exposed which helps maintain the hydrophilicity of the composite over time. This leads to improved osteointegration and sustained growth of new bone tissue.

The composites of the invention have an open cell structure. This allows infiltration of new bone cells into the matrix to promote growth of new bone.

Due to the presence of the dispersed CaP, the composites of the invention have a higher surface area than do similar porous polymer matrices made without incorporation of CaP. This indicates that at least some of the nanosized CaP is exposed on the surface of the polymer matrix and is contributing to the surface area of the composite. Suitable surface areas for the composite are in the range of 5-50 m²/g, preferably 7 to 50 m²/g.

The stiffness of the composite may be controlled by controlling the relative amounts of polymer and CaP used. Increasing the amount of CaP relative to the polymer will increase the stiffness of the composite but will tend to make it more brittle. Lowering the amount of CaP relative to the amount of polymer will increase the flexibility but decrease the stiffness of the composite.

The hydrophilicity of the composite may also be controlled by controlling the relative amounts of polymer and CaP used, with hydrophilicity increasing as the amount of CaP used increases.

Suitable weight ratios of CaP to polymer include about 1:4 to about 3:1, preferably 1:3 to about 1:1, preferably about 1:2. At ratios above 1:1, for example 2CaP:1PCL, the composites become brittle and harder to handle.

In one embodiment, the composites of the invention do not contain any NaCl.

Once formed, the composite may be ground into particles, preferably of about 0.1 to about 1 mm in diameter, and the particles suspended in a pharmaceutically acceptable solvent. The resulting suspension or paste can then be injected directly into an injury site to promote healing. Suitable pharmaceutically acceptable solvents for injection include glycerol, water, propylene glycol and PEG-12. Composites containing ratios of CaP to polymer of above 1:1 are particularly suitable for grinding into particles.

### Methods

The process of the invention is defined in the appended claims. Herein described is a first process for the preparation of a porous hydrophilic composite, the process comprising:
(a) mixing a solution of a biodegradable polymer in a first solvent with nanosized calcium phosphate (CaP), preferably with a dispersion of nanosized CaP in a second solvent, until the CaP is homogeneously distributed throughout the resulting mixture;
(b) solidifying the mixture of step (a) to form a gel; and
(c) removing the first and second solvents from the gel of step (b) by washing with a third solvent to leave a porous hydrophilic composite containing the nanosized CaP dispersed throughout a porous polymer matrix.

The preferred features of the porous composites discussed above apply equally to the first process and the products thereof.

Step (a) involves mixing a solution of a biodegradable polymer in a first solvent with nanosized calcium phosphate (CaP), preferably with a dispersion of nanosized calcium phosphate (CaP) in a second solvent, until the CaP is homogeneously distributed throughout the resulting mixture.

The first solvent should be one in which the polymer can be fully dissolved but where the solubility is highly temperature dependent over a convenient temperature range (such as between about 0 °C and 50 °C). For example, preferred are first solvents in which the polymer has limited solubility at temperatures below about 10 °C, such as below about 5 °C. Preferably, the first solvent is one in which the polymer can be fully dissolved in the first solvent at ambient temperate (20 °C) or with warming, for example on warming to 50 °C or less, such as 40-45 °C, within a convenient time period. Dissolution of the polymer can be facilitated by stirring or other agitation of the solvent.

Suitably, the minimum volume of first solvent required to dissolve the polymer is used, both for cost reasons and to facilitate later solvent removal. The amount of first and second solvents should also not be so high that a gel cannot be formed in step (b). The amount of solvent used must however be balanced against the desired degree of porosity of the final product as the total volume of first and second solvents used will influence the porosity of the final composite, with higher solvent volumes tending to lead to more porous composites.

If added in powder form, nanosized CaP particles tend to agglomerate and are difficult to disperse in the polymer solution. The CaP is therefore preferably used as a dispersion in a second solvent. For example, the nanosized CaP may be synthesised using a method which results directly in formation of a suitable dispersion. Preferably, the dispersion of CaP in the second solvent does not contain anything other than the CaP and the second solvent.

The first and second solvents may be the same or different, but are preferably the same. If the first and second solvents are different, the second solvent should be miscible with the first and should not adversely influence the solubility of the polymer.

If the polymer is a water insoluble polymer such as PCL, PLA or PGA, suitable first solvents include tetrahydrofuran (THF), dioxane, ethyl acetate and acetone, with acetone being preferred. Suitable second solvents for use when the polymer is water insoluble include THF, dioxane, ethyl acetate, acetone and mixtures thereof, with acetone being preferred.

If the polymer is a water soluble polymer such as poly(vinyl alcohol) (PVA), soluble collagen, hyaluronic acid, gelatine or chitosan, suitable first and second solvents include water, ethanol, methanol and iso-propyl alcohol (IPA), with water being preferred.

Preferred are first and second solvents which are biocompatible, so that any solvent residue remaining in the porous composite does not pose a health risk when the composite is used *in vivo.* For this reason, acetone is a preferred first and second solvent when water insoluble polymers are used, and water is a preferred first and second solvent when water soluble polymers are used.

The solution of the biodegradable polymer in the first solvent and the nanosized calcium phosphate (CaP), such as the dispersion of nanosized calcium phosphate (CaP) in the second solvent, should be mixed until the CaP is homogeneously distributed throughout the resulting mixture. This may take about an hour. Homogeneous distribution of the CaP in the mixture may be assessed visually.

Once the mixing is complete and the CaP is homogeneously distributed throughout the mixture. the mixture may be cooled down until the mixture starts to solidify to form a gel. Conveniently, this can be done by allowing the mixture to cool to room temperature (if required) and then placing it in a refrigerator at about 4-5 °C. Alternatively, the mixture may be placed directly in a refrigerator. Lower temperatures may also be used, for example 0 °C or below, but these are less convenient from a processing standpoint.

Alternatively, the mixture may also be cooled by pouring into a mold which is at a lower temperature than the mixture. For example, the mixture may be poured into a pre-cooled mold. Solidification to form a gel may then be completed by placing the filled mold in a refrigerator. Molds may be pre-cooled by placing in a refrigerator or freezer prior to use.

In one embodiment, step (b) of the first method preferably comprises cooling the mixture of step (a) to solidify it.

As the mixture starts to solidify, it forms a gel incorporating the CaP and the first and second solvents. Particularly when forming large composite structures where the solidification takes time, the gel that is being formed may be regularly agitated (e.g. by stirring or shaking) to avoid sedimentation of the CaP particles. This helps ensure a homogenous distribution of the CaP in the resulting composite material.

Once a gel starts to form, the entire mixture may be transferred to a filter to facilitate the later washing step (c).

Once solidification of the mixture is substantially complete, the resulting gel is washed with a third solvent to remove the first and second solvents. Removal of the first and second solvents from the solidified gel results in pores in the polymer matrix.

Solidification of the mixture can be assessed by seeing whether or not the mixture will flow when the container holding it is tipped. Alternatively, if small waves form on the surface of the mixture when the side of the container is tapped gently, this indicates that the mixture has not yet solidified.

The third solvent is different to the first and second solvents. The third solvent should be miscible with the first and second solvents, such that they dissolve in the third solvent and are removed by the washing. The third solvent should not be a solvent for the polymer.

When the polymer is water insoluble, suitable third solvents include water, IPA, ethanol and methanol. Preferably, the third solvent is IPA or water, more preferably water. Some shrinkage of the gel may occur during the washing step, with water leading to less shrinkage than IPA.

When the polymer is water soluble, suitable third solvents include THF, dioxane, ethyl acetate, ethylene glycol, glycerol and acetone, with acetone being preferred.

If amorphous HA is used as the CaP, use of water as the third solvent may lead to conversion to crystalline HA. To avoid this, methanol is a preferred third solvent when the CaP is amorphous HA.

As the third solvent is not a solvent for the polymer, washing step (c) may also help promote final solidification of the polymer.

Removal of the first and second solvents from the gel in washing step (c) leaves a behind a porous polymer matrix with nanosized CaP distributed throughout.

In a preferred embodiment, the first and second solvents are both acetone and the third solvent is water. More preferably, the polymer is PCL, the first and second solvents are both acetone and the third solvent is water

Traces of water left in the porous composite may render it susceptible to unwanted microbial growth. Therefore, if the third solvent is water, after step (c), the resulting porous solid composite may be washed with a fourth solvent to remove traces of water.

The fourth solvent should be miscible with but more volatile than water, such that traces of the fourth solvent can be easily removed by drying the resulting composite. It should also not be a solvent for the polymer. Suitable fourth solvents include MeOH, EtOH and iso-propyl alcohol (IPA), preferably IPA. IPA is a preferred fourth solvent as it is easy to remove by drying at 40 °C, it is not very toxic if small amounts were to be left in the composite, and it is not very hygroscopic when compared to methanol and ethanol which always contain small amounts of water and so can leave traces of moisture in the structure.

The polymer cooling and solidification step (step (b)) may take place in a mold to control the shape of the resulting composite. Once the polymer has solidified sufficiently, the composite can be removed from the mold and immersed in the third solvent to remove the first and second solvents. If the third solvent is water, the resulting porous solid molded composite may be subsequently immersed in a fourth solvent as defined above to remove traces of water.

Voids can also be introduced into the composite by solidifying the composite around a removable support, which when removed leaves voids in the composite corresponding to the shape and size of the removable support. For example, the composite may be moulded around a removable pin array. Following solidification of the composite, the pin array may be removed, leaving voids in the composite corresponding to the shape and size of the pin array. Such voids may take the form of channels or holes in the composite material have a longest dimension of about 5 to 40 mm and a width or diameter of about 0.25 to 2 mm.

The processes of the invention have a number of advantages over prior art processes to form bone scaffolds. For example, the use of strong acids which may dissolve the CaP and/or cause polymer degradation is avoided. Heating to melt the polymer is not required, and suitable choice of the first solvent avoids the need for excessive heating to dissolve the polymer. Cooling below 0 °C to form a gel is also not normally necessary.

The mixture formed in step (a) may also be deposited using a 3D printer. This can allow complex shaped composites to be built up layer by layer. The mixture can be ejected from a nozzle which is immersed in water or other suitable third solvent, depending on the nature of the polymer. The movement of the nozzle is controllable in the x-y-z directions, which allows for a controlled layer-by-layer build up of the composite structure.

### Implant forming

The composites of the present invention may be used as scaffolds for the promotion of new bone growth. In some embodiments, the composite may be combined with a load-bearing, non-degradable structure, which may also be referred to as an implant.

The preferred features of the porous composites and the methods discussed above apply equally to this aspect.

In an alternative process embodiment, a substrate may be dipped into the mixture formed from the biodegradable polymer, the first solvent, the nanosized calcium phosphate (CaP) and the second solvent and then removed. A thin layer of the mixture will be left on the surface of the substrate, which after solidification of the polymer and removal of the first and second solvents will result in a thin layer of composite present as a coating on the substrate surface. The dipping step may be repeated to build up multiple layers of composite on the substrate surface.

Following the dipping step and solidification of the polymer, the solvent removal may be carried out by dipping the coated substrate in the third solvent. Alternatively, the first and second solvents may be removed by evaporation.

It has been found that using a third solvent to remove the first and second solvents in step (c) of the first method leads to a high degree of porosity in the resulting composite, such as about 50% to about 90%. If the first and second solvents are instead allowed to evaporate, the voids in the polymer network left by the evaporating solvent(s) tend to collapse such that the polymer chains can aggregate and form a denser structure, for example with a maximum porosity of only 10-20%.

The thickness and porosity of the layer on the substrate surface may therefore be controlled by choice of the method used to remove the first and second solvents. If the first and second solvents are removed using a third solvent, as in the first method disclosed, a porous layer will result. Alternatively, if the first and second solvents are removed by evaporation, a denser (i.e. less porous) and thinner layer will result.

A composite layer formed by dipping may be from about 400 nm to about 100 micrometers in thickness, for example 500 nm to 50 micrometers thick.

Herein disclosed is a second process for the preparation of a hydrophilic composite for promoting bone growth, the process comprising:
(a1) mixing a solution of a biodegradable polymer in a first solvent with a dispersion of nanosized calcium phosphate (CaP) in a second solvent until the CaP is homogeneously distributed throughout the resulting mixture;
(b1) solidifying the mixture of step (a) on a substrate to form a gel; and
(c1) removing the first and second solvents from the gel of step (b) by evaporation to leave a layer of a porous hydrophilic composite containing the nanosized CaP dispersed throughout a polymer matrix on the surface.

The preferred features of the porous composites and the first method discussed above apply equally to the second process and the products thereof.

Pre-cooling of the substrate, for example to 4-5 °C, before the dipping step can help initiate solidification of the polymer onto the substrate surface.

Preferably, when a composite is prepared by dipping a substrate into the mixture, the first and second solvents are both acetone and the polymer is PCL.

In another embodiment of the first process, at least a portion of a substrate may be fully or partially immersed in the mixture formed in step (a) before the polymer solidifies. The polymer will then solidify around the substrate. After removal of the first and second solvents, a scaffold containing some or all of the substrate embedded within the porous composite will be formed.

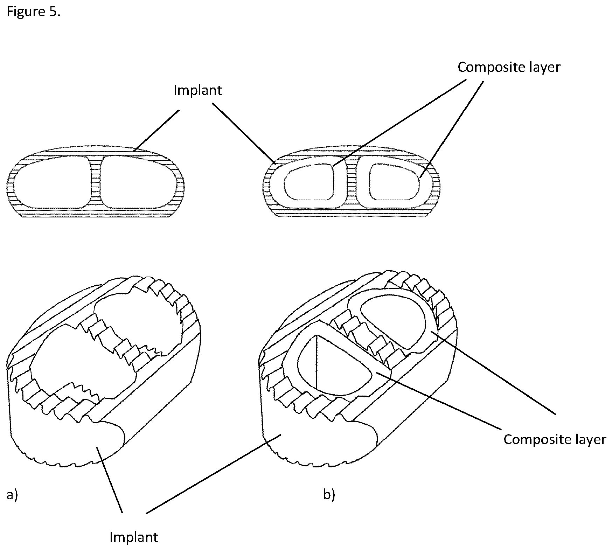

In this embodiment, a substrate can be introduced into the mixture in any suitable vessel, for example a mold. Alternatively, the substrate may be placed in a mold and the mixture then introduced into the mold. If the substrate is placed in a mold, the overall shape of the resulting scaffold will be controlled by the shape of the mold. In addition, formation of composite can be directed to surfaces of the substrate which are not in contact with the mold. For example, Figure 5b shows the formation of composite on the inner surface of a spinal cage. This was produced by placing the case in a suitably shaped Teflon mold in which the outer surface of the cage was in contact with the mold, thus excluding it from composite formation.

In one embodiment, step (b) of the first method can therefore comprise solidifying the polymer on or around a substrate.

Optionally, one or more layers of composite may be deposited on the substrate surface by dipping, before immersion of the coated substrate in the mixture. This may allow formation of layers of composite of different density and porosity depending on the method used for removal of the first and second solvents.

A denser layer will generally adhere more strongly to the underlying substrate, whereas a more porous layer will better promote bone growth. In one preferred embodiment, a first layer is formed by allowing the first and second solvents to evaporate and a second and any subsequent layers is/are formed by washing to remove the first and second solvents.

Suitable substrates for use in the invention include orthopaedic and dental implants, including but not limited to screws, spinal fusion cages, wires, meshes, nails, pins, rods, plates, hip stems, ostomy bag ports, bone anchored hearing aids, and dental implant abutments.

Suitable substrates include, but are not limited to, substrates made from metals such as titanium and its alloys, zirconium and its alloys, stainless steel, tantalum, NiTi alloys and cobalt-chrome alloys; ceramics such as alumina, zirconia, alumina toughened zirconia, and Si3N4; graphitic material such as graphene and pyrocarbon; and polymers such as polypropylene, polyethylene, polysulfone (PSU), polyether ketone (PEKK), poly(styrene), poly(carbonate), poly(ethylene terephthalate) and PEEK. Preferred substrates include titanium and its alloys, zirconium and its alloys, stainless steel, tantalum, NiTi alloys, cobalt-chrome alloys, alumina, zirconia, alumina toughened zirconia, pyrocarbon, polysulfone (PSU), polyether ketone (PEKK), poly(styrene), poly(carbonate) and PEEK. More preferred substrates include titanium and its alloys, stainless steel, zirconia, alumina toughened zirconia, pyrocarbon, and PEEK, most preferably titanium or PEEK.

The hydrophilicity of the porous hydrophilic composites formed by the above methods may be further increased by treating the composites with a biocompatible organic polyol. The processes of the invention comprise, after step (c) or step (c1);
(d) immersing the porous hydrophilic composite in a solution comprising a biocompatible organic polyol;
(e) removing the porous hydrophilic composite from the solution; and
(f) drying the porous hydrophilic composite.

The organic polyol should be biocompatible. Suitable organic polyols include glycerol, propylene glycol, polyvinyl alcohol or low molecular weight polyethylene glycols such as PEG-12. Suitable polyethylene glycols are those with a molecular weight of about 200-2000 g/mol. A preferred polyol is glycerol.

The polyol is applied to the composites by immersing the composite in a solution of the polyol in a volatile organic solvent. By "volatile organic solvent" is meant a solvent which is more volatile than the polyol. Suitable volatile organic solvents are non-toxic. The solvent should also be completely miscible with the organic polyol. If the polyol is a solid, it should be soluble in the solvent. The solvent should not be a solvent for the polymer in the polymer matrix.

Suitable volatile organic solvents include isopropanol and ethanol. A suitable solution contains about 0.25 - 50 wt. % of the organic polyol, for example about 0.25 to 50 wt.% of glycerol in isopropanol. Preferably, the mixture contains at least 0.5 wt. % glycerol in isopropanol.

Suitably, the composite is immersed in the solution for up to 10 minutes, more preferably up to 5 minutes. Generally, the composite will float in the solution initially and will then sink as solution displaces air and fills the pores in the composite. The pores of the composite have been filled with the solution when the composite sinks and there are no longer any visible air bubbles being released from the composite.

After removal of the composite from the solution, it is dried to remove the volatile organic solvent. Drying can involve leaving the composite at room temperature to allow the volatile organic solvent to evaporate. The treated composite may also be heated to speed up evaporation of the volatile organic solvent. The heating temperature should not be high enough to evaporate the polyol or damage the polymer matrix. Suitable temperatures for such heating are in the range of about 30 to 40 °C, preferably about 35 °C. The removal of the volatile solvent can be monitored by weighing the composite during the heating. Once the volatile solvent has been removed, any increase in weight of the treated composite compared to the starting composite before immersion can be attributed to the polyol.

Without being bound by theory, it is postulated that the polyol is adsorbed on at least a portion of an internal and/or external surface of the composite and/or forms a coating on at least a portion of an internal and/or external surface thereof, thereby increasing the hydrophilicity. By "internal surfaces of the composite" is meant the surfaces of any pores or voids within the composite.

The present invention provides a porous hydrophilic composite for promoting bone growth, as described herein, wherein the porous hydrophilic composite further comprises from about 0.5 to 50 wt% of a biocompatible organic polyol. The wt.% is based on the total weight of the composite and the biocompatible organic polyol. The biocompatible organic polyol may be adsorbed on at least a portion of an internal and/or external surface of the porous hydrophilic composite and/or forms a coating on at least a portion of an internal and/or external surface thereof.

Higher concentrations of biocompatible organic polyol will improve the hydrophilicity, and will also make the composite softer and less brittle/friable.

The following examples are non-limiting illustrations of the invention.

### EXAMPLES

### Chemicals

Ethylene glycol (anhydrous, 99 %), isopropanol (99.5 %), glycerol (anhydrous, 99%), CaO, H₃PO₄ (85 %) and poly(caprolactone) 80 000 g/mol (PCL) were obtained from Sigma Aldrich, Sweden.

### Analytical instruments

A Zeiss FEG-SEM Sigma 300 with Gemini optics was used for the SEM analysis. For the XRD analysis, a Bruker D8 instrument with CuKα radiation (1.54 Å) was used. A Micromeritics TriStar instrument was used for the nitrogen adsorption analysis.

### Example 1. Synthesis of porous HA / PCL composite (reference example)

### 1a) Synthesis of HA gel

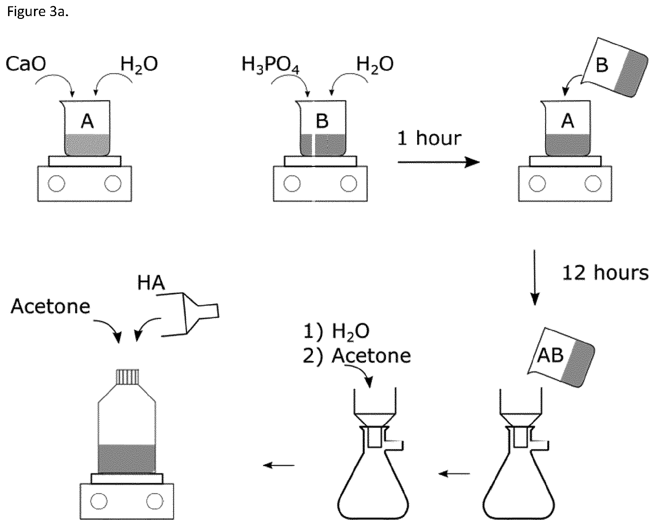

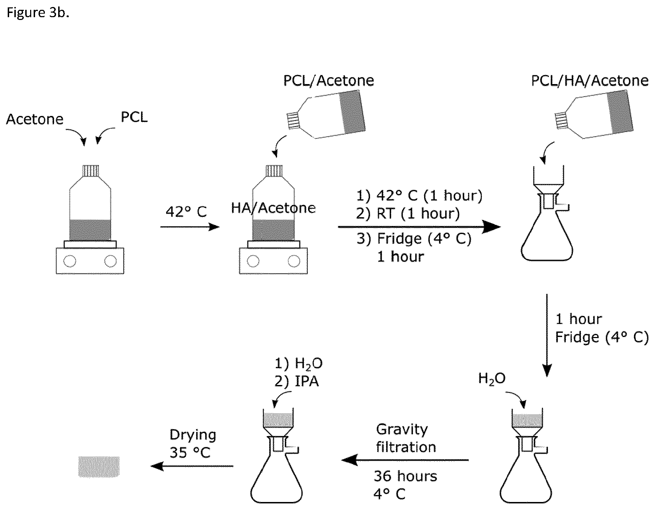

A schematic of the process can be found in Figures 3a and 3b. 2.82 g of powdered CaO was mixed with 150 ml of H₂O in a beaker and the resulting dispersion was stirred for 1 hour. In a separate beaker, 3.48 g of H₃PO₄ (85 wt.%) was mixed with 150 ml of H₂O. The contents of the two beakers were mixed at ambient temperature, and the resulting gel was stirred for 12 hours. The gel was then filtered in a grade 4 glass filter and washed extensively with 1 L of water and subsequently 1 L of acetone. The product was a gel containing nanosized HA (approximately 5 g) and acetone (approximately 25 ml).

### 1b) Analysis of HA gel sample

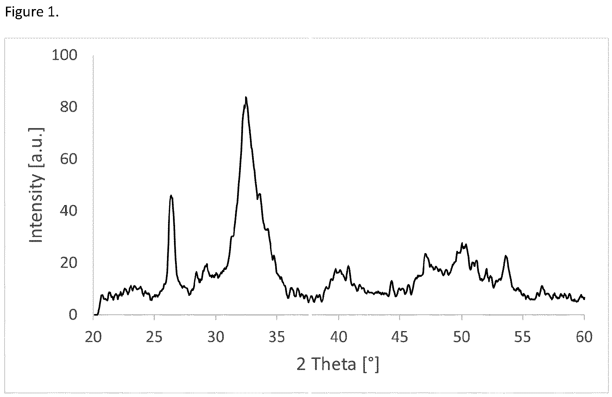

A portion of the gel from Step 1a) was dried and analysed using XRD and nitrogen adsorption. The XRD analysis (Figure 1) showed that the sample consisted of crystalline HA. The specific surface area, as determined using the Brunauer - Emmet -Teller (BET) method, of this sample was found to be 200 m²/g.

### 1c) Production of foam

The acetone / CaP gel (with a total weight of approximately 25 g) prepared in Step 1a) was transferred to a bottle and 75 ml of acetone was added (to give a total acetone volume of approximately 100 ml). The resulting mixture was stirred for 12 hours. In a separate bottle, 10 g of PCL was mixed with 80 ml of acetone, and the bottle was heated to 42 °C for 12 hours under stirring to dissolve the PCL. The PCL / acetone mixture was then added to the acetone / CaP gel mixture and allowed to stir for approximately 1 hour at 42 °C. Stirring was then continued as the mixture was cooled to room temperature. It was then placed in a refrigerator at 4-5 °C for 1 hour, with shaking approximately every 5 minutes. The resulting HA / acetone / PCL gel was placed in a glass filter which had been cooled to 4 °C. Upon pouring the HA / acetone / PCL gel into the glass filter, the mixture immediately solidified, creating a solid HA / acetone / PCL foam. After allowing the foam to fully solidify at 4 °C, a process which took approximately 60 minutes, the foam and glass filter was placed on a Büchner flask at room temperature. The foam was washed with 1 L of water and subsequently with 1 L of isopropanol. The resulting solid foam was dried at 35 °C for 12 hours to provide a PCL / HA composite.

### 1d) Analysis of foam

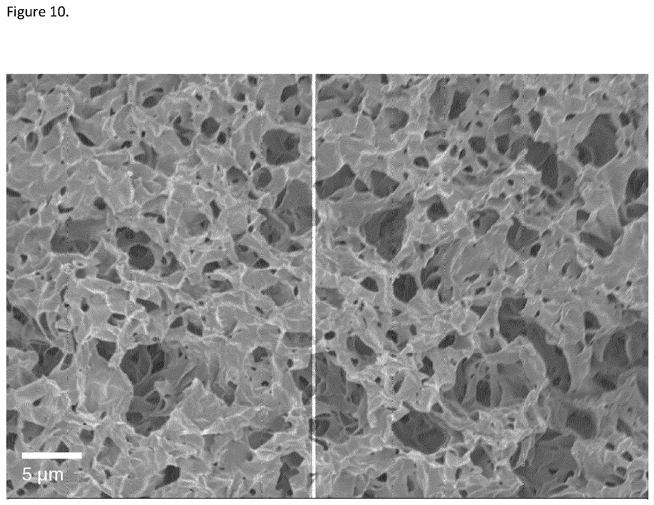

A sample of the foam produced in Step 1c) was sputtered with gold and analysed using SEM. A representative image is shown in Figure 10. As seen from this image, the pores of the foam are between 0.25-10 µm in size. It can also be seen that there are no large agglomerates of HA visible and that the HA crystals are evenly dispersed in the polymer matrix.

A portion of the foam was also analysed with nitrogen adsorption (BET). Analysis of the foam gave a specific surface area of 22.0 m²/g. As a comparison, a pure PCL foam was also synthesized. This was done using the procedure described in Step 1c), without any addition of CaP/acetone and using a total amount of 160 ml of acetone (an extra amount of 80 ml acetone was added to compensate for the extra amount that would otherwise come from the acetone / CaP gel). The pure PCL foam had a specific surface area of 6.2 m²/g.

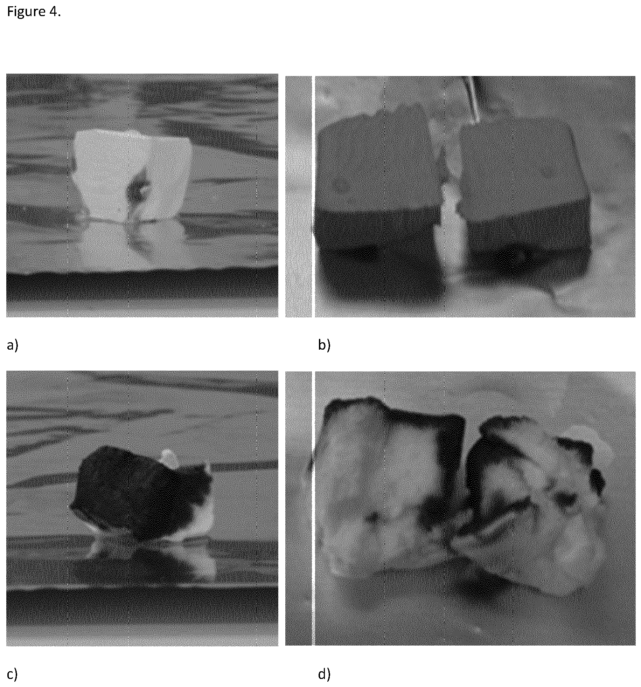

The porous pure PCL foam prepared according to Example 1d and the composite prepared according to Example 1c were immersed in toluidine blue. The more hydrophilic the composite, the greater the degree of infiltration of the toluidine blue into the interior of the structure. Figure 4 shows the results of this test, with Figure 4a showing the outside of the foam and Figure 4b showing the inside of the foam of Example 1d after immersion, and Figures 4c and 4d showed the outside and inside respectively of the composite of Example 1c after immersion. As can be seen from a comparison of Figures 4a and 4b with Figures 4c and 4d, the composite of the invention was more hydrophilic than the pure PCL foam.

### Example 2. Synthesis of porous ACP / PCL composite using ethylene glycol (reference example)

### 2a) Synthesis of ACP gel

135 g of ethylene glycol and 2.82 g of CaO were mixed with 15 ml of H₂O in a beaker. In a separate beaker, 135 g of ethylene glycol and 3.48 g of H₃PO₄ were mixed with 15 ml of H₂O. The contents of each beakers were stirred for 30 minutes separately. Then, the contents of the two beakers were mixed at ambient temperature, and the resulting gel was stirred for 24 hours. The gel was filtered in a grade 4 glass filter and washed extensively with 2 L of isopropanol and subsequently with 1 L of acetone. The product was a gel containing nanosized ACP (approximately 5 g) and acetone (approximately 25 ml).

### 2 b) Analysis of gel sample

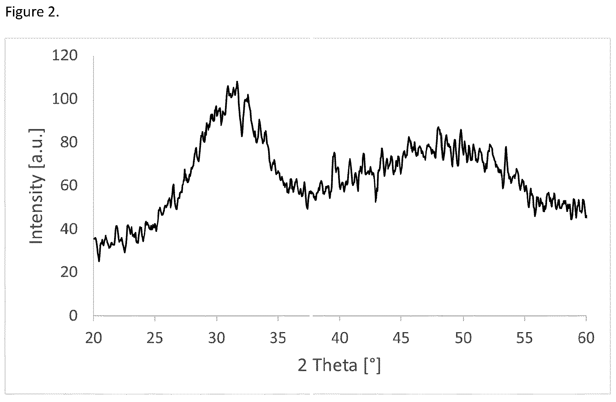

A sample of the gel produced in Step 2a) was dried and analysed with XRD and nitrogen adsorption (BET). The XRD result showed (Figure 2) that amorphous calcium phosphate was obtained. The specific surface area, as determined using the BET method, was 315 m²/g.

### 2c) Production of foam

The acetone / CaP gel prepared in Step 2a) was transferred to a bottle and 75 ml of acetone was added. The resulting mixture was stirred for 12 hours. In a separate bottle, 10 g of PCL was mixed with 80 ml of acetone, and the bottle was heated to 42 °C for 12 hours under stirring to dissolve the PCL. The PCL / acetone mixture was then transferred to the acetone / CaP gel mixture and allowed to stir for approximately 1 hour at 42 °C. Stirring was then continued as the mixture was cooled to room temperature. It was then placed in a refrigerator at 4-5 °C for 1 hour, with shaking approximately every 5 minutes. The resulting ACP / acetone / PCL gel was placed in a glass filter which had been cooled to 4 °C. Upon pouring the ACP / acetone / PCL gel into the glass filter, the mixture immediately solidified, creating a solid ACP / acetone / PCL foam. After allowing the foam to fully solidify at 4 °C, a process which took approximately 60 minutes, the foam and glass filter was placed on a Büchner flask at room temperature. The foam was washed with 1 L of methanol and subsequently with 1 L of isopropanol. The resulting solid foam was dried at 35 °C for 12 hours.

### Example 3. Synthesis of porous HA / PCL composite using ethylene glycol (reference example)

### 3a) Synthesis of HA

135 g of ethylene glycol, and 2.82 g of CaO were mixed with 15 ml of H₂O in a beaker. In a separate beaker, 135 g of ethylene glycol and 3.48 g of H₃PO₄ were mixed with 15 ml of H₂O. The contents of the beakers were stirred for 30 minutes separately. Then the contents of the two beakers were mixed at ambient temperature. 1000 ml of water was poured into the mixture. The mixture was stirred for 3 days and the resulting gel material was filtered in a grade 4 glass filter and washed extensively with 2 L of acetone. The product was a gel containing nanosized HA (approximately 5 g) and acetone (approximately 25 ml).

### 3b) Analysis of gel sample

A sample of the gel produced in Step 3a was dried and analysed with XRD and nitrogen adsorption (BET). The XRD result showed that nanosized hydroxyapatite was obtained. The specific surface area, as calculated with the BET method, was 320 m²/g.

### 3c) Production of foam

The mixture produced in Step 3a) was processed according to Example 2, Step c) to produce a foam

### Example 4. Synthesis of porous ACP / PCL composite using glycerol (reference example)

This composite was produced exactly as described in Example 2, but with the ethylene glycol being replaced with glycerol. The specific surface area of the ACP powder was measured as 318 m²/g.

### Example 5. Production of a HA/PCL coating on a glass surface (reference example)

A CaP / acetone / PCL mixture was prepared according to Example 1a-1c, but instead of cooling the CaP / acetone / PCL mixture to 4 °C, the mixture was kept at 42 °C. Glass slides used for microscopy (O. Kindler GmbH, 26 x 76 mm) were dipped into the mixture and removed. The acetone was then allowed to evaporate from the slides, resulting in a thin and homogenous CaP / PCL film on the surface of the slides.

The adhesion strength of the CaP / PCL film to the glass surface was assessed using the Scotch^{®} tape test using Scotch^{®} tape (810, 3M). The test was conducted by placing Scotch^{®} tape on CaP / PCL coated microscopy slides, followed by applying a load (1 kg) for approximately 30 seconds, after which the tape was removed and the amount of CaP / PCL film present on the tape as well as on the glass slide was qualitatively evaluated by means of visual inspection. The results showed that the CaP / PCL film was not significantly affected by the Scotch^{®} tape test, i.e. the CaP / PCL film was well adhered to the substrate.

### Example 6. In situ formation of a HA / PCL composite in a PEEK spinal cage implant (reference example)

A CaP / acetone / PCL mixture was prepared according to Example 1a-1c, but instead of cooling the CaP / acetone / PCL mixture to 4 °C, the mixture was kept at 42 °C. A PEEK cage (illustrated in Figure 5a) was placed in a Teflon mold which had inserts that were shaped to the same form as the inside channels as the PEEK cage, but with a gap of 2 mm between the mold and PEEK cage channels. The Teflon mold and PEEK cage were cooled down to 4 °C. The CaP / acetone / PCL mixture was poured into the gap between the cage and mold, and the mixture was allowed to solidify for 60 minutes. The cage and mold were placed in water for 60 minutes, and the mold was carefully removed, leaving a thin layer of composite on the inside channels of the PEEK cage (illustrated in Figure 5b). The PEEK cage was flushed with isopropanol and left to dry at room temperature.

### Example 7. In situ formation of a CaP / PCL composite in titanium lattice (reference example)

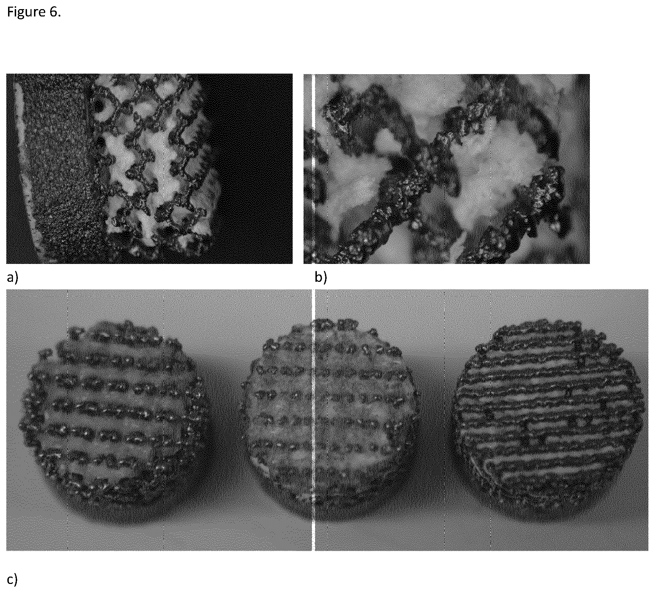

A porous titanium substrate, pre-cooled to 4 °C, with a truss length of 2.5 mm was completely immersed in a mixture of CaP / acetone / PCL (prepared as Example 1a-1c, but instead of cooling the CaP / acetone / PCL mixture to 4 °C, the mixture was kept at 42 °C) followed by placement at 4 °C in order to induce solidification. After approximately 60 minutes, excess CaP / Acetone / PCL foam surrounding the structure was removed. The acetone was replaced by immersing the substrate in a beaker with 100 ml water for 10 minutes, followed by immersion in a beaker with 100 ml of isopropanol for 10 minutes, and subsequent drying at room temperature. The resulting product was a porous structure with CaP / PCL foam penetrating into the titanium structure and filling the pores in the titanium substrate (see Figures 6a and 6b).

This procedure was tested for different titanium porous structures, and these samples were then immersed in a 1 wt. % solution of alizarin red, which selectively stains for calcium. The samples were then washed in water to remove excess alizarin red. As shown in Figure 6c, the structures were completely red stained, which shows that the calcium phosphate is evenly distributed in the polymer matrix.

### Example 8. In situ formation of CaP / PCL composite to produce a coating on an implant screw (reference example)

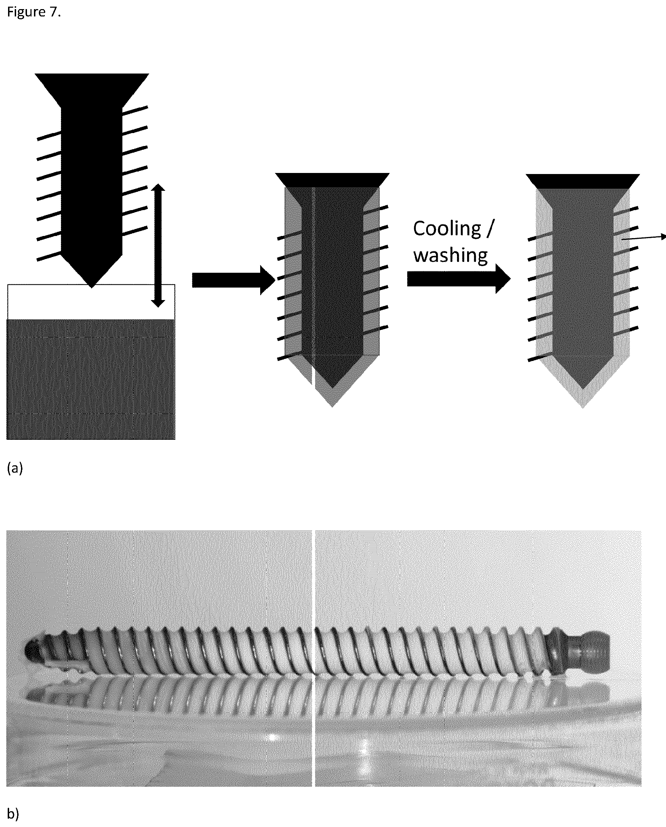

A CaP / acetone / PCL mixture was prepared according to Example 1a-1c, but instead of cooling the CaP / acetone / PCL mixture to 4 °C, the mixture was kept at 42 °C instead. A pedicle screw for spinal fusion surgery was dipped in the mixture and then placed in water for 10 minutes, which created a thin coating of CaP / PCL foam. The screw was then placed in isopropanol for 10 minutes and allowed to dry in room temperature. An image of the screw is shown in Figure 7.

### Example 9. Implantation of porous HA / PCL composite in rabbit calvaria (reference example)

A HA/PCL foam was prepared as described in Example 1. Cylindrical plugs, 5 mm in diameter and 6 mm high, were punched out from the foam using an appropriate tool. The plugs were sterilized using E-Beam radiation (5 KGy). Holes of 6 mm diameter were drilled in into rabbit calvaria. Sterilized foam plugs were then implanted in some of the holes whilst some were left unfilled as a control (sham) in order to assess the ability of the HA / PCL foam composite to induce bone formation. The holes, with and without implants, were allowed to heal for 6 weeks. The animals were then euthanized and the areas were imaged with micro-CT. Samples were subsequently processed for histomorphometry.

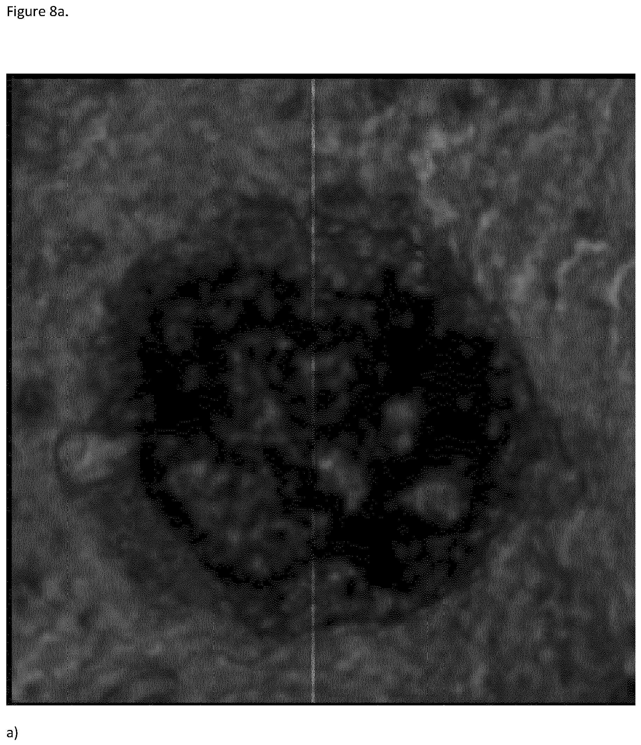

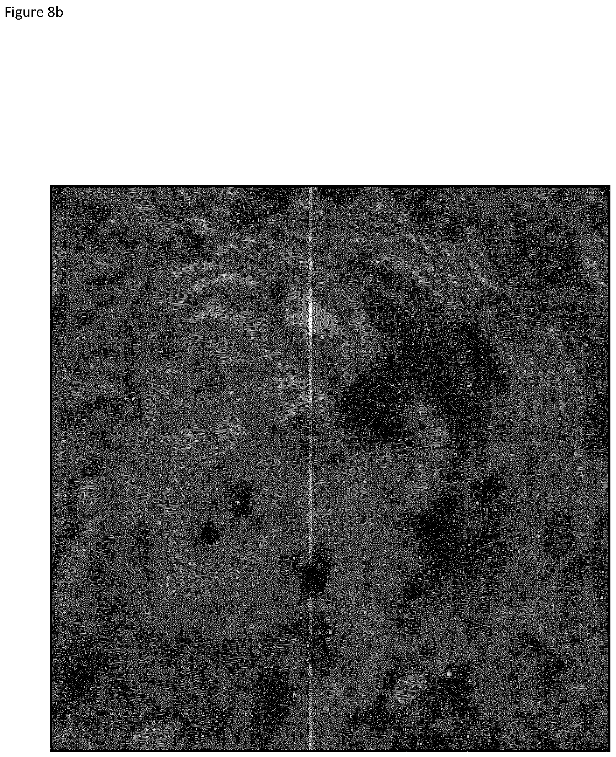

Figure 8 shows a micro-CT image of a control sham (image a) and a hole which had a foam plug inserted (image b). As can be seen from image b, the composite has been completely overgrown with new bone whereas the sham showed little or no new bone growth. This illustrates that the porous composites of the invention can promote bone growth in defects that are too large to heal naturally.

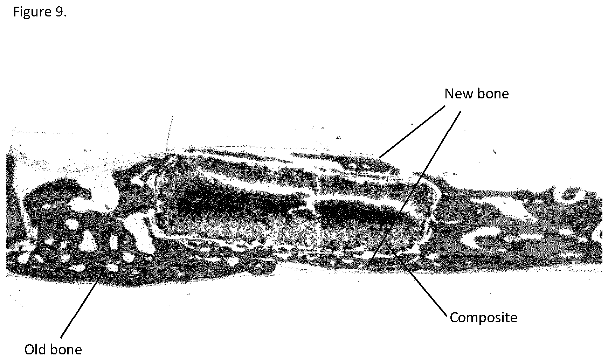

Figure 9 shows a histology slide of the sample in Figure 8b. This slide confirms that the composite is overgrown with a thin layer of new bone. Formation of new bone was also observed on the underside of the composite. The irregular shape along the edges of the composite shows that the composite has partially biodegraded during the implantation period.

### Example 10. Formation of regularly distributed pores in the composite (reference example)

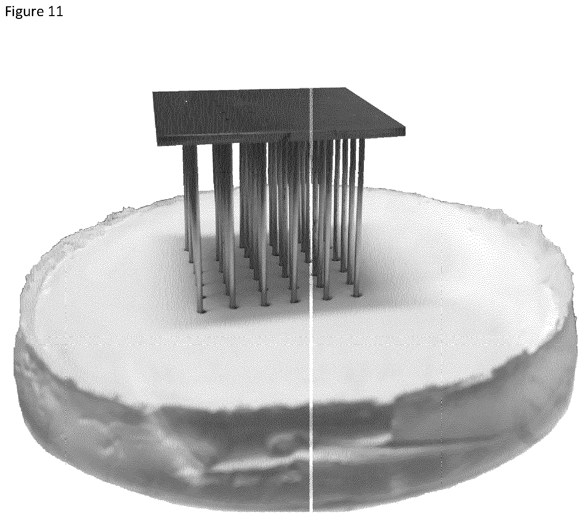

A foam was produced according to Example 1c. A pin array with regularly distributed pins, 0.9 mm in diameter, 30 mm long and with a spacing between the pins of 5 mm, made of 316 stainless steel, was placed in the foam (15 g) after the water washing procedure, as shown in Figure 11. The foam was then washed with isopropanol, the pin array was removed and the foam was allowed to dry at 35 °C for 12 hours. The result was a foam composite with regularly distributed cylindrical channels, each having a diameter of 0.9 mm.

### Example 11. Improvement of hydrophilicity of the composite using 1,2,3-Propanetriol (glycerol)

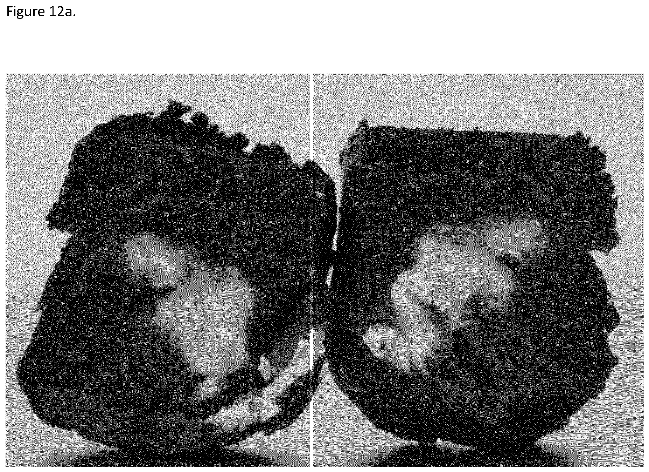

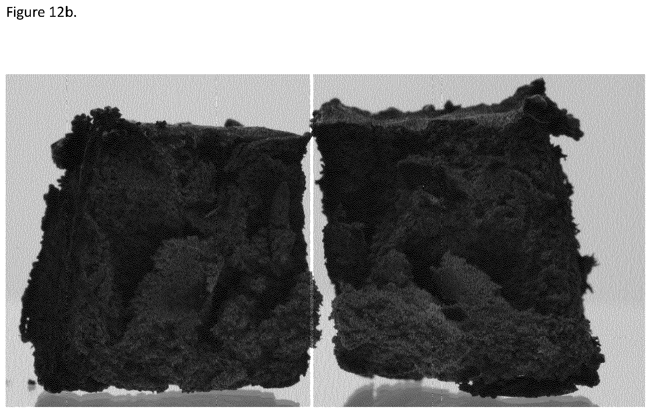

A foam batch was produced according to Example 1c. A mixture of 0.5 wt. % glycerol in isopropanol was prepared. Approximately 1 g of foam was immersed in 50 mL glycerol / isopropanol mixture for 30 minutes. The foam piece was then removed from the solution and dried at 35 °C for 12 hours. The foam piece was then immersed in azorubine dye, 1 wt. % in Type-1 water, for 30 minutes, after which a photograph of a cross section of the foam was taken (Figure 12b). A second foam piece synthesized according to Example 1c, without any glycerol / isopropanol treatment, was also immersed in red dye for 30 minutes after which a photograph of a cross section of the foam was taken (Figure 12a). The inner parts of the untreated foam piece were not reached by the dye (the white areas in Figure 12a). For the glycerol / isopropanol treated foam piece, the dye had completely wetted the whole foam piece (Figure 12b), indicating that the glycerol treatment had increased the hydrophilicity and hence the wettability of the foam by the dye.

### Example 12. Production of HA / PCL composites with different CaP / PCL ratios (reference example)

Foam batches were produced according to Example 1c, but with varying CaP /PCL weight ratios, in addition to the 33.3:66.7 CaP / PCL weight ratio which is described in Example 1c. A qualitative description of the properties of the different composites is as follows:
0 CaP / 100 PCL: formed a flexible and soft foam, which was easy to deform and was hydrophobic. The porosity was approximately 90 %.
10 CaP / 90 PCL: had properties similar to 0 CaP / 100 PCL. Hydrophobic.
20 CaP / 80 PCL: soft and flexible. Easy to deform. Hydrophilic. The porosity was 85-90 %.
33.3 CaP / 66.7 PCL: soft and flexible, harder than 20 CaP / 80 PCL. Hydrophilic.
50 CaP / 50 PCL: was hard and somewhat brittle. Very hydrophilic. The composite was divided into granules with a scalpel and mixed with water to form a moldable, voluminous putty. The porosity was approximately 80 %.
75 CaP / 25 PCL: was extremely hard and brittle. Formed a dense structure, with a porosity of 60-70 %. Very hydrophilic. This composite was crushed to granules in a mortar, the granules were then mixed with 50 wt. % glycerol to form an injectable paste.

## Claims

1. A porous hydrophilic composite for promoting bone growth, the composite comprising:
(a) a porous biodegradable polymer matrix,
(b) nanosized calcium phosphate (CaP) homogeneously dispersed throughout the polymer matrix, wherein the CaP has a specific surface area in the range of about 180 to about 380 m²/g, and
(c) from about 0.5 to 50 wt% of a biocompatible organic polyol, based on the total weight of the composite including the biocompatible organic polyol;
with the proviso that the composite does not contain any copolymer(s) of lactic acid and glycolic acid

2. The porous hydrophilic composite of claim 1, wherein the nanosized CaP is amorphous calcium phosphate (ACP), beta-TCP, calcium deficient HA (CDHA) or hydroxyapatite (HA)

3. The porous hydrophilic composite of claim 1, wherein the CaP is nanocrystalline.

4. The porous hydrophilic composite of claim 1 or claim 2, wherein the CaP is amorphous.

5. The porous hydrophilic composite of any preceding claim, wherein the CaP has a specific surface area in the range of about 200 to about 350 m²/g, and/or wherein the porous hydrophilic composite has a specific surface area in the range of about 5 to about 50 m²/g, and/or wherein the ratio of CaP to polymer matrix is about 1:4 to about 3:1 by weight.

6. The porous hydrophilic composite of any preceding claim, wherein the polymer matrix comprises or consists of poly(caprolactone) (PCL), poly(lactic acid) (PLA), poly(glycolic acid) (PGL), poly(vinyl alcohol) (PVA), soluble collagen, hyaluronic acid, glycerine or chitosan, preferably wherein the polymer matrix comprises or consists of poly(caprolactone) (PCL).

7. The porous hydrophilic composite of any preceding claim, wherein the organic polyol is adsorbed on at least a portion of an internal and/or external surface thereof and/or forms a coating on at least a portion of an internal and/or external surface thereof.

8. The porous hydrophilic composite of any preceding claim, wherein the organic polyol is a polyethylene glycol with a molecular weight in the range of about 200 g/mol to about 2,000 g/mol, glycerol, propylene glycol or polyvinyl alcohol, preferably wherein the organic polyol is glycerol.

9. A scaffold for use in bone reconstruction, the scaffold comprising a substrate and the porous hydrophilic composite of any preceding claim.

10. The scaffold of claim 9, wherein the substrate is selected from orthopaedic and dental implants, preferably wherein the substrate is selected from screws, spinal fusion cages, wires, meshes, nails, pins, rods, plates, hip stems, ostomy bag ports, bone anchored hearing aids, and dental implant abutments; and/or
wherein the substrate is made from metal, ceramic, graphitic material or a polymer; and/or wherein the substrate is made from titanium and its alloys, stainless steel, zirconia, alumina toughened zirconia, pyrocarbon, or PEEK, preferably titanium or PEEK.

11. A process for the preparation of the porous hydrophilic composite of any of claims 1-8, the process comprising:
(a) mixing a solution of a biodegradable polymer in a first solvent with nanosized calcium phosphate (CaP), preferably with a dispersion of nanosized calcium phosphate (CaP) in a second solvent, until the CaP is homogeneously distributed throughout the resulting mixture;
(b) solidifying the mixture of step (a) to form a gel;
(c) removing the first and second solvents from the gel of step (b) by:
(i) washing with a third solvent to leave a porous hydrophilic composite containing the nanosized CaP homogeneously dispersed in a porous polymer matrix, or
(ii) evaporation to leave a layer of a porous hydrophilic composite containing the nanosized CaP dispersed throughout a polymer matrix on the surface;
(d) immersing the porous hydrophilic composite in a solution comprising a biocompatible organic polyol;
(e) removing the porous hydrophilic composite from the solution; and
(f) drying the porous hydrophilic composite.

12. The process of claim 11, wherein the third solvent is IPA or water, preferably water.

13. The process of any of claims 11 or 12, wherein the first and second solvents are the same, and/or
wherein the first and second solvents are independently selected from THF, dioxane and acetone, preferably wherein the first and second solvents are both acetone.

14. The process of any of claims 11 to 13, wherein the CaP has a specific surface area in the range of about 180 to about 380 m²/g, preferably wherein the CaP has a specific surface area in the range of about 200 to about 350 m²/g.

15. An injectable formulation comprising a dispersion of particles of the porous hydrophilic composite of any of claims 1 to 8 and a pharmaceutically acceptable solvent, preferably wherein the pharmaceutically acceptable solvent is selected from glycerol, water for injection, propylene glycol and PEG-12.

## Patentansprüche

1. Poröse hydrophile Verbundmaterialien zur Förderung des Knochenwachstums, wobei die Verbundmaterialien Folgendes umfassen:
(a) eine poröse biologisch abbaubare Polymermatrix,
(b) Calciumphosphat (CaP) in Nanogröße, das homogen über die gesamte Polymermatrix verteilt ist, wobei das CaP einen spezifischen Oberflächenbereich in der Größenordnung von etwa 180 bis etwa 380 m²/g aufweist, und
(c) von etwa 0,5 bis 50 Gew.-% eines biokompatiblen organischen Polyols bezogen auf das Gesamtgewicht der Verbundmaterialien einschließlich des biokompatiblen organischen Polyols;
mit der Maßgabe, dass die Verbundmaterialien kein(e) Polymer(e) von Milchsäure und Glykolsäure enthalten

2. Poröse hydrophile Verbundmaterialien nach Anspruch 1, wobei das CaP in Nanogröße amorphes Calciumphosphat (ACP), beta-TCP, calciumarmes HA (CDHA) oder Hydroxyapatit (HA) ist.

3. Poröse hydrophile Verbundmaterialien nach Anspruch 1, wobei das CaP nanokristallin ist.

4. Poröse hydrophile Verbundmaterialien nach Anspruch 1 oder Anspruch 2, wobei das CaP amorph ist.

5. Poröse hydrophile Verbundmaterialien nach einem vorstehenden Anspruch, wobei das CaP einen spezifischen Oberflächenbereich in der Größenordnung von etwa 200 bis etwa 350 m²/g aufweist und/oder wobei die porösen hydrophilen Verbundmaterialien einen spezifischen Oberflächenbereich in der Größenordnung von etwa 5 bis etwa 50 m²/g aufweisen und/oder wobei das Verhältnis von CaP zu Polymermatrix bezogen auf das Gewicht etwa 1:4 bis etwa 3:1 beträgt.

6. Poröse hydrophile Verbundmaterialien nach einem vorstehenden Anspruch, wobei die Polymermatrix Poly(caprolacton) (PCL), Poly(milchsäure) (PLA), Poly(glykolsäure) (PGL), Poly(vinylalkohol) (PVA), lösliches Kollagen, Hyaluronsäure, Glycerin oder Chitosan umfasst oder daraus besteht, wobei die Polymermatrix vorzugsweise Poly(caprolacton) (PCL) umfasst oder daraus besteht.

7. Poröse hydrophile Verbundmaterialien nach einem vorstehenden Anspruch, wobei das organische Polyol an mindestens einem Abschnitt einer Innen- und/oder Außenfläche davon adsorbiert wird und/oder eine Beschichtung auf mindestens einem Abschnitt einer Innen- und/oder Außenfläche davon bildet.

8. Poröse hydrophile Verbundmaterialien nach einem vorstehenden Anspruch, wobei das organische Polyol ein Polyethylenglykol in der Größenordnung von etwa 200 g/mol bis etwa 2.000 g/mol, Glyzerol, Propylenglykol oder Polyvinylalkohol ist, wobei das organische Polyol vorzugsweise Glyzerol ist.

9. Gerüst zur Verwendung bei der Knochenrekonstruktion, wobei das Gerüst ein Substrat und die porösen hydrophilen Verbundmaterialien nach einem vorstehenden Anspruch umfasst.

10. Gerüst nach Anspruch 9, wobei das Substrat aus orthopädischen und Zahnimplantaten ausgewählt ist, wobei das Substrat vorzugsweise aus Schrauben, Wirbelsäulenfusionskäfigen, Drähten, Netzen, Nägeln, Stiften, Stangen, Platten, Hüftschaften, Stomabeutelanschlüssen, knochenverankerten Hörgeräten und Zahnimplantataufbauten ausgewählt ist; und/oder
wobei das Substrat aus Metall, Keramik, graphithaltigem Material oder einem Polymer gefertigt ist; und/oder
wobei das Substrat aus Titan und seinen Legierungen, rostfreiem Stahl, Zirkonoxid, mit Aluminiumoxid gehärtetem Zirkonoxid, Pyrokohlenstoff oder PEEK, vorzugsweise Titan oder PEEK gefertigt ist.

11. Prozess zur Herstellung der porösen hydrophilen Verbundmaterialien nach einem der Ansprüche 1-8, wobei der Prozess Folgendes umfasst:
(a) Mischen einer Lösung aus einem biologisch abbaubaren Polymer in einem ersten Lösungsmittel mit Calciumphosphat (CaP) in Nanogröße, vorzugsweise mit einer Dispersion aus Calciumphosphat (CaP) in Nanogröße in einem zweiten Lösungsmittel, bis das CaP über die gesamte resultierende Mischung homogen verteilt ist;
(b) Erhärten der Mischung aus Schritt (a) zum Bilden eines Gels;
(c) Entfernen des ersten und zweiten Lösungsmittels aus dem Gel von Schritt (b) durch:
(i) Waschen mit einem dritten Lösungsmittel, um poröse hydrophile Verbundmaterialien, die das CaP in Nanogröße enthalten, in einer porösen Polymermatrix homogen verteilt zurückzulassen, oder
(ii) Evaporieren, um eine Schicht von porösen hydrophilen Verbundmaterialien, die das CaP in Nanogröße enthalten, über eine gesamte Polymermatrix homogen auf der Oberfläche verteilt zurückzulassen;
(d) Eintauchen der porösen hydrophilen Verbundmaterialien in eine Lösung, die ein biokompatibles organisches Polyol umfasst;
(e) Entfernen der porösen hydrophilen Verbundmaterialien aus der Lösung; und
(f) Trocknen der porösen hydrophilen Verbundmaterialien.

12. Prozess nach Anspruch 11, wobei das dritte Lösungsmittel IPA oder Wasser, vorzugsweise Wasser ist.

13. Prozess nach einem der Ansprüche 11 oder 12, wobei das erste und das zweite Lösungsmittel gleich sind, und/oder
wobei das erste und das zweite Lösungsmittel unabhängig voneinander aus THF, Dioxan und Azeton ausgewählt sind, wobei das erste und das zweite Lösungsmittel vorzugsweise beide Azeton sind.

14. Prozess nach einem der Ansprüche 11 bis 13, wobei das CaP einen spezifischen Oberflächenbereich in der Größenordnung von etwa 180 bis etwa 380 m²/g aufweist, wobei das CaP vorzugsweise einen spezifischen Oberflächenbereich in der Größenordnung von etwa 200 bis etwa 350 m²/g aufweist.

15. Einspritzbare Formulierung, umfassend eine Dispersion von Partikeln der porösen hydrophilen Verbundmaterialien nach einem der Ansprüche 1 bis 8 und ein pharmazeutisch verträgliches Lösungsmittel, wobei das pharmazeutisch verträgliche Lösungsmittel vorzugsweise aus Glyzerol, Wasser zum Einspritzen, Propylenglykol und PEG-12 ausgewählt ist.

## Revendications

1. Composite hydrophile poreux destiné à favoriser la croissance osseuse, le composite comprenant :
(a) une matrice polymère biodégradable poreuse,
(b) du phosphate de calcium (CaP) nanométrique dispersé de manière homogène dans toute la matrice polymère, dans lequel le CaP présente une surface spécifique située dans la plage comprise entre environ 180 et environ 380 m²/g, et
(c) environ 0,5 à 50 % en poids d'un polyol organique biocompatible, sur la base du poids total du composite incluant le polyol organique biocompatible ;
à condition que le composite ne contienne aucun copolymère d'acide lactique et d'acide glycolique.

2. Composite hydrophile poreux selon la revendication 1, dans lequel le CaP nanométrique est du phosphate de calcium amorphe (ACP), du bêta-TCP, de l'HA déficitaire en calcium (CDHA) ou de l'hydroxyapatite (HA).

3. Composite hydrophile poreux selon la revendication 1, dans lequel le CaP est nanocristallin.

4. Composite hydrophile poreux selon la revendication 1 ou la revendication 2, dans lequel le CaP est amorphe.

5. Composite hydrophile poreux selon une revendication précédente quelconque, dans lequel le CaP présente une surface spécifique située dans la plage comprise entre environ 200 et environ 350 m²/g, et/ou dans lequel le composite hydrophile poreux présente une surface spécifique située dans la plage comprise entre environ 5 et environ 50 m²/g, et/ou dans lequel le rapport entre le CaP et la matrice polymère est compris entre environ 1:4 et environ 3:1 en poids.

6. Composite hydrophile poreux selon une revendication précédente quelconque, dans lequel la matrice polymère comprend ou est constituée de poly(caprolactone) (PCL), poly(acide lactique) (PLA), poly(acide glycolique) (PGL), poly(alcool vinylique) (PVA), collagène soluble, acide hyaluronique, glycérine ou chitosane, de préférence dans lequel la matrice polymère comprend ou est constituée de poly(caprolactone) (PCL).

7. Composite hydrophile poreux selon une revendication précédente quelconque, dans lequel le polyol organique est adsorbé sur au moins une partie d'une surface interne et/ou externe de celui-ci et/ou forme un revêtement sur au moins une partie d'une surface interne et/ou externe de celui-ci.

8. Composite hydrophile poreux selon une revendication précédente quelconque, dans lequel le polyol organique est un polyéthylène glycol avec un poids moléculaire situé dans la plage comprise entre environ 200 g/mol et environ 2 000 g/mol, du glycérol, du propylène glycol ou de l'alcool polyvinylique, de préférence dans lequel le polyol organique est du glycérol.

9. Structure destinée à être utilisée dans la reconstruction osseuse, la structure comprenant un substrat et le composite hydrophile poreux selon une revendication précédente quelconque.

10. Structure selon la revendication 9, dans laquelle le substrat est sélectionné parmi des implants orthopédiques et dentaires, de préférence dans laquelle le substrat est sélectionné parmi des vis, des cages de spondylodèse, des fils, des mailles, des clous, des broches, des tiges, des plaques, des tiges de hanche, des ports de poche de stomie, des prothèses auditives à ancrage osseux et des piliers d'implants dentaires ; et/ou
dans laquelle le substrat est constitué de métal, de céramique, d'un matériau graphitique ou d'un polymère ; et/ou
dans laquelle le substrat est constitué de titane et de ses alliages, d'acier inoxydable, de zircone, de zircone renforcée à l'alumine, de pyrocarbone, ou de PEEK, de préférence de titane ou de PEEK.

11. Procédé pour la préparation du composite hydrophile poreux selon l'une quelconque des revendications 1 à 8, le procédé comprenant :
(a) un mélange d'une solution d'un polymère biodégradable dans un premier solvant avec du phosphate de calcium (CaP) nanométrique, de préférence avec une dispersion de phosphate de calcium (CaP) nanométrique dans un deuxième solvant, jusqu'à ce que le CaP soit réparti de manière homogène dans tout le mélange obtenu ;
(b) une solidification du mélange de l'étape (a) pour former un gel ;
(c) le fait d'enlever les premier et deuxième solvants du gel de l'étape (b) par :
(i) lavage avec un troisième solvant pour laisser un composite hydrophile poreux contenant le CaP nanométrique dispersé de manière homogène dans une matrice polymère poreuse, ou
(ii) évaporation pour laisser une couche d'un composite hydrophile poreux contenant le CaP nanométrique dispersé dans toute une matrice polymère à la surface ;
(d) une immersion du composite hydrophile poreux dans une solution comprenant un polyol organique biocompatible ;
(e) le fait d'enlever le composite hydrophile poreux de la solution ; et
(f) un séchage du composite hydrophile poreux.

12. Procédé selon la revendication 11, dans lequel le troisième solvant est de l'IPA ou de l'eau, de préférence de l'eau.

13. Procédé selon l'une quelconque des revendications 11 ou 12, dans lequel les premier et deuxième solvants sont identiques, et/ou
dans lequel les premier et deuxième solvants sont indépendamment sélectionnés parmi le THF, le dioxane et l'acétone, de préférence dans lequel les premier et deuxième solvants sont tous deux de l'acétone.

14. Procédé selon l'une quelconque des revendications 11 à 13, dans lequel le CaP présente une surface spécifique située dans la plage comprise entre environ 180 et environ 380 m²/g, de préférence dans lequel le CaP présente une surface spécifique située dans la plage comprise entre environ 200 et environ 350 m²/g.

15. Formulation injectable comprenant une dispersion de particules du composite hydrophile poreux selon l'une quelconque des revendications 1 à 8 et un solvant pharmaceutiquement acceptable, de préférence dans laquelle le solvant pharmaceutiquement acceptable est sélectionné parmi le glycérol, l'eau pour injection, le propylène glycol et le PEG-12.
